Europäisches Patentamt

European Patent Office

Office européen des brevets

(11) Veröffentlichungsnummer: **0 231 774**
**B1**

# (12) EUROPÄISCHE PATENTSCHRIFT

(45) Veröffentlichungstag der Patentschrift:
**18.04.90**

(51) Int. Cl.⁴: **C07C 51/41**, C07C 51/43,
B01D 7/00

(21) Anmeldenummer: **87100390.1**

(22) Anmeldetag: **14.01.87**

(54) Verfahren zur Herstellung von reinen Hydroxylammoniumsalzen von Fettsäuren mit 1 bis 4 Kohlenstoffatomen.

(30) Priorität: **17.01.86 DE 3601216**

(43) Veröffentlichungstag der Anmeldung:
**12.08.87 Patentblatt 87/33**

(45) Bekanntmachung des Hinweises auf die Patenterteilung:
**18.04.90 Patentblatt 90/16**

(84) Benannte Vertragsstaaten:
**BE CH DE FR GB IT LI NL**

(56) Entgegenhaltungen:
EP-A- 0 108 294
US-A- 2 483 252

**GMELINS HANDBUCH DER ANORGANISCHEN CHEMIE, 8. Auflage, System Nr. 23, Lieferung 2, Seite 599; Verlag Chemie, Weinheim**

(73) Patentinhaber: **BASF Aktiengesellschaft,
Carl-Bosch-Strasse 38, D-6700 Ludwigshafen(DE)**

(72) Erfinder: **Fuchs, Hugo, Dr., Egellstrasse 28,
D-6700 Ludwigshafen(DE)**
Erfinder: **Weiss, Franz-Josef, Dr., Schilfweg 1,
D-6708 Neuhofen(DE)**

## Beschreibung

Aus der US-PS 2 483 252 ist ein Verfahren zur Herstellung von Hydroxylammoniumsalzen niederer Fettsäuren bekannt, bei dem man Hydroxylammoniumchlorid oder -sulfat mit Alkalisalzen von niederen Fettsäuren in alkoholischer Lösung bei erhöhter Temperatur umsetzt. Aus dieser Lösung wird zunächst nach Abkühlen Alkalisulfat bzw. -chlorid auskristallisiert und abgetrennt und anschließend die alkoholische Lösung von Hydroxylammoniumsalzen von niederen Fettsäuren eingedampft und letztere durch Kristallisation gewonnen. Dieses Verfahren hat den Nachteil, daß die erhaltenen Hydroxylammoniumsalze von niederen Fettsäuren nicht frei von Fremdionen sind, da durch die Kristallisation eine Verunreinigung nur schwer auszuschließen ist. Andererseits ist eine weitere Reinigung durch fraktionierte Kristallisation sehr aufwendig.

Es war deshalb die technische Aufgabe gestellt, ein Verfahren zur Herstellung von reinen Hydroxylammoniumsalzen von niederen Fettsäuren zur Verfügung zu stellen, das auf einfache Weise die Verunreinigung des erzeugten Wertproduktes durch Fremdionen ausschließt.

Diese Aufgabe wird gelöst in einem Verfahren zur Herstellung von reinen Hydroxylammoniumsalzen von Fettsäuren mit 1 bis 4 Kohlenstoffatomen durch Umsetzen von Hydroxylammoniumsulfat mit Alkalisalzen von Fettsäuren in Lösung bei erhöhter Temperatur und Abtrennen der Hydroxylammoniumsalze von Fettsäuren mit 1 bis 4 Kohlenstoffatomen, dadurch gekennzeichnet, daß man

a) Hydroxylammoniumsulfat mit Alkalisalzen von Fettsäuren mit 1 bis 4 Kohlenstoffatomen in wäßriger Lösung oder gelöst in Alkanolen mit 1 bis 3 Kohlenstoffatomen oder Mischungen derselben bei einer Temperatur von 20 bis 70°C umsetzt,

b) aus dem so erhaltenen Umsetzungsgemisch das Lösungsmittel verdampft und einen trockenen, d.h. lösungsmittelfreien Rückstand erhält,

c) aus dem so erhaltenen trockenen Rückstand die Hydroxylammoniumsalze von Fettsäuren mit 1 bis 4 Kohlenstoffatomen durch Sublimation unter vermindertem Druck gewinnt.

Das neue Verfahren hat den Vorteil, daß man Hydroxylammoniumsalze von Fettsäuren mit 1 bis 4 Kohlenstoffatomen von hoher Reinheit erhält. Ferner hat das neue Verfahren den Vorteil, daß es wenig aufwendig ist.

Erfindungsgemäß geht man von Hydroxylammoniumsulfat aus. Vorteilhaft hat das verwendete Hydroxylammoniumsulfat einen Gehalt an Ammoniumionen von <500ppm, insbesondere <100ppm. Solches Hydroxylammoniumsulfat erhält man vorteilhaft durch mehrmaliges, z.B. 2- bis 3-maliges Kristallisieren aus wäßriger Lösung. Vorteilhaft verfährt man hierbei so, daß man jeweils die wäßrige Lösung von Hydroxylammoniumsulfat bei einer Temperatur von 48 bis 80°C unter einem Druck von 50 bis 400 mbar eindampft, abkühlt und kristallisiertes Hydroxylammoniumsulfat gewinnt. Durch mehrmalige Wiederholung der Kristallisation gelingt es, den Ammoniumionengehalt des Hydroxylammoniumsulfats auf den vorgenannten Wert zu bringen.

Als zweiten Reaktionspartner verwendet man Alkalisalze von Fettsäuren mit 1 bis 4 Kohlenstoffatomen. Bevorzugte Alkalisalze sind Natrium- oder Kaliumsalze, insbesondere Natriumsalze der genannten Säuren. Geeignete Ausgangsstoffe sind beispielsweise Natriumformiat, Natriumacetat, Kaliumacetat, Natriumpropionat oder Kaliumbutyrat. Die Salze können wasserfrei vorliegen oder Kristallwasser gebunden enthalten. Bevorzugt verwendet man Alkalisalze der Essigsäure. Demzufolge ist Hydroxylammoniumacetat ein bevorzugtes Verfahrenserzeugnis.

Die Umsetzung wird in alkoholischer oder wäßriger Lösung oder Gemischen derselben durchgeführt. Geeignete Alkanole mit 1 bis 3 Kohlenstoffatomen sind z.B. Methanol, Ethanol oder Propanol. Besonders bevorzugt wird Methanol verwendet.

Die Ausgangsstoffe werden zweckmäßig als alkoholische oder wäßrige Lösung gemischt. Zweckmäßig haben die verwendeten Lösungen ein Gehalt von 10 bis 50 Gew.% an Hydroxylammoniumsulfat bzw. an Alkalisalzen der genannten Fettsäuren. Es ist auch möglich, einen Reaktionspartner, z.B. Hydroxylammoniumsulfat gelöst vorzulegen und Alkalisalze der genannten Fettsäuren in feiner Verteilung und fester Form in die Lösung einzutragen oder umgekehrt. Eine weitere Verfahrensvariante besteht darin, daß man eine Lösung von Hydroxylammoniumsulfat vorlegt und in dieser Alkalisalze der genannten Fettsäuren in situ erzeugt, indem man die gewünschte Fettsäure, z.B. Essigsäure zugibt und diese mit der äquivalenten Menge an Natronlauge neutralisiert.

Hydroxylammoniumsulfat und Alkalisalze der genannten Fettsäure werden vorteilhaft im Molverhältnis von 1:1 angewandt. Es hat sich bewährt, wenn man die Umsetzung bei einer Temperatur von 20 bis 70°C, insbesondere 40 bis 60°C durchführt.

Erfindungsgemäß wird aus dem so erhaltenen Reaktionsgemisch, das nunmehr Hydroxylammoniumsalze der genannten Fettsäuren sowie Alkalisulfat in dem verwendeten Lösungsmittel enthält, das Lösungsmittel verdampft, bis man einen trockenen Rückstand erhält. Vorteilhaft hält man beim Verdampfen eine Temperatur von 5 bis 60°C ein. Falls der Siedepunkt des verwendeten Lösungsmittels höher liegt, führt man das Verdampfen des Lösungsmittels unter vermindertem Druck, z.B. von 5 bis 500 mbar durch.

Aus dem so erhaltenen trockenen Rückstand, der aus festen Hydroxylammoniumsalzen der genannten Fettsäuren und Alkalisulfat besteht, werden die jeweils enthaltenen Hydroxylammoniumsalze der Fettsäuren durch Sublimation unter vermindertem Druck gewonnen. Vorteilhaft führt man die Sublimation bei einer Temperatur von 10 bis 50°C und unter einem Druck von 0,1 bis 200 mbar durch.

Nach dem erfindungsgemäßen Verfahren gelingt es, Hydroxylammoniumsalze von Fettsäuren mit einer Reinheit >99 % herzustellen. Hydroxylammoniumacetat, das nach der Erfindung erhältlich ist, eignet sich beispielsweise zur Umsetzung von Carbonylverbindungen in organischen Lösungsmitteln.

Das Verfahren nach der Erfindung sei aus folgendem Beispiel veranschaulicht.

Beispiel 1

Herstellung von Hydroxylammoniumacetat

82 g (0,5Mol) Hydroxylammoniumsulfat mit einem Gehalt an Ammoniumionen von 100 ppm werden mit 100 g Wasser vermischt. Hierzu werden bei 40°C 136 g Natriumacetat-Trihydrat in 100 g Wasser hinzugefügt. Der pH-Wert der Lösung beträgt 5,6. Die Lösung wird anschließend bei ca. 50°C im Vakuum im Rotationsverdampfer bis zur Trockne eingedampft. Als Eindampfrückstand erhält man 160 g. Das Reaktionsgemisch wird nun in eine Sublimationsapparatur überführt und bei 40°C und 0,5 mbar sublimiert. Als Sublimat erhält man 91 g Hydroxylammoniumacetat mit einer Reinheit von 99,5 %. Der nicht sublimierbare Rückstand besteht aus Natriumsulfat mit Spuren von Hydroxylammoniumacetat.

Beispiel 2

Herstellung von Hydroxylammoniumpropionat

74 g Propionsäure werden mit 40 g Natronlauge in 180 ml Wasser in das Natriumsalz der Propionsäure überführt. Anschließend fügt man 82 g Hydroxylammoniumsulfat mit einem Gehalt an Ammoniumionen <500 ppm in 100 ml Wasser bei einer Temperatur von 40°C hinzu. Die Lösung wird wie in Beispiel 1 entwässert. Dabei erhält man einen Eindampfrückstand von 174 g. Dieser wird bei 40°C und 0,5 mbar sublimiert. Als Sublimat werden 101 g Hydroxylammoniumpropionat mit einer Reinheit von 99,2% erhalten.

**Patentansprüche**

1. Verfahren zur Herstellung von reinen Hydroxylammoniumsalzen von Fettsäuren mit 1 bis 4 Kohlenstoffatomen durch Umsetzen von Hydroxylammoniumsulfat mit Alkalisalzen von Fettsäuren mit 1 bis 4 Kohlenstoffatomen in Lösung bei erhöhter Temperatur und Abtrennen der Hydroxylammoniumsalze von Fettsäuren mit 1 bis 4 Kohlenstoffatomen, dadurch gekennzeichnet, daß man
   a) Hydroxylammoniumsulfat und Alkalisalze von Fettsäuren mit 1 bis 4 Kohlenstoffatomen gelöst in Wasser oder Alkanolen mit 1 bis 3 Kohlenstoffatomen oder Gemischen derselben bei einer Temperatur von 20 bis 70°C umsetzt,
   b) aus dem so erhaltenen Reaktionsgemisch das Lösungsmittel verdampft und einen trockenen Rückstand erhält,
   c) aus dem so erhaltenen trockenen Rückstand die Hydroxylammoniumsalze von Fettsäuren mit 1 bis 4 Kohlenstoffatomen durch Sublimation unter vermindertem Druck gewinnt.
2. Verfahren nach Anspruch 1, dadurch gekennzeichnet, daß man Wasser oder Methanol oder Gemisch derselben als Lösungsmittel verwendet.
3. Verfahren nach den Ansprüchen 1 und 2, dadurch gekennzeichnet, daß man das Lösungsmittel in der Stufeb bei einer Temperatur von 5 bis 60°C und unter einem Druck von 5 bis 500 mbar verdampft.
4. Verfahren nach den Ansprüchen 1 bis 3, dadurch gekennzeichnet, daß man in der Stufe c bei der Sublimation eine Temperatur von 10 bis 50°C und einen Druck von 0,1 bis 200 mbar einhält.
5. Verfahren nach den Ansprüchen 1 bis 4, dadurch gekennzeichnet, daß man Hydroxylammoniumsulfat und Natriumacetat-Trihydrat als Ausgangsstoffe verwendet.

**Claims**

1. A process for preparing a pure hydroxylammonium salt of a fatty acid of 1 to 4 carbon atoms by reacting hydroxylammonium sulfate with an alkali metal salt of a fatty acid of 1 to 4 carbon atoms in solution at elevated temperature and separating off the hydroxylammonium salt of the fatty acid of 1 to 4 carbon atoms, which comprises
   a) reacting hydroxylammonium sulfate and an alkali metal salt of a fatty acid of 1 to 4 carbon atoms in solution in water or an alkanol of 1 to 3 carbon atoms or a mixture thereof at 20–70°C,
   b) evaporating the solvent out of the resulting reaction mixture to obtain a dry residue,
   c) isolating from the dry residue thus obtained the hydroxylammonium salt of the fatty acid of 1 to 4 carbon atoms by sublimation under reduced pressure.
2. A process as claimed in claim 1, wherein the solvent used is water, methanol or a mixture thereof.
3. A process as claimed in either of claims 1 and 2, wherein the solvent is evaporated in stage b at 5–60°C and 5–500 mbar.
4. A process as claimed in any one of claims 1 to 3, wherein the sublimation in stage c is carried out at 10–50°C and 0.1–200 mbar.
5. A process as claimed in any one of claims 1 to 4, wherein the starting materials used are hydroxylammonium sulfate and sodium acetate trihydrate.

**Revendications**

1. Procédé de préparation de sels d'hydroxylammonium purs d'acides gras ayant de 1 à 4 atomes de carbone par réaction de sulfate d'hydroxylammonium avec des sels alcalins d'acides gras ayant de 1 à 4 atomes de carbone en solution à températures élevées, et séparation des sels d'hydroxylammonium d'acides gras ayant de 1 à 4 atomes de carbone, caractérisé en ce que
   a) on fait réagir du sulfate d'hydroxylammonium et des sels alcalins d'acides gras ayant de 1 à 4 atomes de carbone en solution dans de l'eau ou des alcanols de 1 à 3 atomes de carbone ou leurs mélanges, à une température de 20 à 70°C;
   b) on évapore le solvant du mélange reactionnel ainsi obtenu et on obtient un résidu séché,
   c) on obtient les sels d'hydroxylammonium des acides gras ayant de 1 à 4 atomes de carbone à partir du résidu séché ainsi obtenu par sublimation sous pression réduite.

2. Procédé selon la revendication 1, caractérisé en ce qu'on utilise comme solvant de l'eau ou du méthanol ou leurs mélanges.

3. Procédé selon les revendications 1 ou 2, caractérisé en ce qu'on évapore le solvant dans l'étape b à une température de 5 à 60°C et sous une pression de 5 à 500 mbar.

4. Procédé selon les revendications 1 à 3, caractérise en ce qu'on maintient dans l'étape c pendant la sublimation une température de 10 à 50°C et une pression de 0,1 à 200 mbar.

5. Procédé selon les revendications 1 à 4, caractérisé en ce qu'on utilise comme substances de départ du sulfate d'hydroxylammonium et de l'acétate de sodium trihydraté.